# EUROPEAN PATENT SPECIFICATION

(11) **EP 3 673 896 B1**
(45) Date of publication and mention of the grant of the patent: **12.01.2022**
(21) Application number: 18425109.8
(22) Date of filing: 28.12.2018
(51) Int. Cl.: A61K 9/00, A61K 9/127

(54) **LIPOSOMIAL EYE DROPS SOLUTION AND USES THEREOF FOR THE TREATMENT OF DRY EYE SYNDROME**
LIPOSOMALE AUGENTROPFENLÖSUNG UND VERWENDUNGEN DAVON ZUR BEHANDLUNG DES TROCKENEN AUGENSYNDROMS
SOLUTION DE GOUTTES LIPOSOMIALES POUR LES YEUX ET LEURS UTILISATIONS POUR LE TRAITEMENT DU SYNDROME DES YEUX SECS

(43) Date of publication of application: 01.07.2020
(73) Proprietor: Dr. Rolf Lambert Pharma-Consulting GmbH, 9230 Flawil (CH)
(72) Inventor: Lambert, Rolf, 9230 Flawil (CH); Cavallo, Giovanni, 00122 Lido di Ostia Roma (IT)
(74) Representative: Sarpi, Maurizio

(56) References cited:
- CN-A- 1 850 054
- CN-B- 102 100 665
- MARTA VICARIO-DE-LA-TORRE ET AL: "Novel Nano-Liposome Formulation for Dry Eyes with Components Similar to the Preocular Tear Film", POLYMERS, vol. 10, no. 4, 11 April 2018 (2018-04-11) , page 425, XP055575757, DOI: 10.3390/polym10040425
- ESSA LAIKA ET AL: "Can the optimum artificial tear treatment for dry eye disease be predicted from presenting signs and symptoms?", CONTACT LENS AND ANTERIOR EYE, STOCKTON PRESS, BASINGSTOKE, GB, vol. 41, no. 1, 12 August 2017 (2017-08-12), pages 60-68, XP085333579, ISSN: 1367-0484, DOI: 10.1016/J.CLAE.2017.07.007

## Description

### Technical Field of the Invention

The present invention relates to ophthalmic formulations, kits and uses thereof, wherein an eye drops solution is composed of Liposomes built with non hydrogenated phospholipids containing Linseed oil, Vitamin A Palmitate, Vitamin E TPGS and in water phase Vitamin B12 and Pycnogenol, according to the claims.

### Background of the Invention

Dry eye syndrome is caused by a chronic lack of sufficient lubrication and moisture on the surface of the eye. Consequences of dry eyes range from subtle but constant eye irritation to significant inflammation and even scarring of the front surface of the eye.

Symptoms of dry eyes and dry eye syndrome include:
- Burning sensation Itchy eyes Aching sensations Heavy eyes
- Fatigued eyes
- Sore eyes Dryness sensation
- Red eyes
- Photophobia(light sensitivity)
- Blurred vision

Another common symptom is something called a foreign body sensation - the feeling that grit or some other object or material is "in" your eye.

Watery eyes also can be a symptom of dry eye syndrome.

This is because dryness on the eye's surface sometimes will over-stimulate production of the watery component of your tears as a protective mechanism.

But this "reflex tearing" does not stay on the eye long enough to correct the underlying dry eye condition. In addition to these symptoms, dry eyes can cause inflammation and (sometimes permanent) damage to the surface of the eye.

### What Causes Dry Eye Syndrome?

Dry eyes are caused by a lack of adequate tears. Your tears are a complex mixture of water, fatty oils and mucus. This mixture helps make the surface of your eyes smooth and clear, and it helps protect your eyes from infection.

For some people, the cause of dry eyes is decreased tear production. For others it's increased tear evaporation and an imbalance in the makeup of your tears.

### Decreased tear production

Dry eyes can occur when you're unable to produce enough tears. The medical term for this condition is keratoconjunctivitis sicca. Common causes of decreased tear production include:
- Aging
- Certain medical conditions, including diabetes, rheumatoid arthritis, lupus, scleroderma, Sjogren's syndrome, thyroid disorders and vitamin A deficiency;
- Certain medications, including antihistamines, decongestants, hormone replacement therapy, antidepressants, and drugs for high blood pressure, acne, birth control and Parkinson's disease;
- Laser eye surgery, though symptoms of dry eyes related to this procedure are usually temporary;
- Tear gland damage from inflammation or radiation;

An adequate and consistent layer of tears on the surface of the eye is essential to keep your eyes healthy, comfortable and seeing well.

Tears bathe the eye's surface to keep it moist and wash away dust, debris and microorganisms that could damage the cornea and lead to an eye infection.

A normal tear film consists of three important components:
1. An oily (lipid) component
2. A watery (aqueous) component
3. A mucous-like (mucin) component

### Aim of the invention

To try to solve or at least to reduce in part, the negative effects of dry eyes syndrome as above shortly described the Applicant studied and developed a formulation in which had to be present component that:
have anti inflammatory action;
have antioxidant action ;
restore the oil layer on eye surface;
maintain moisture;
can be sterilized.

Briefly, through different steps, the activity carried out by the Applicant allowed to define how to produce, at industrial scale, an eye drops solution composed of Liposomes built with non hydrogenated phospholipids containing Linseed oil, Vitamin A Palmitate, Vitamin E TPGS, and in water phase Vitamin B12 and Pycnogenols.

This liposomal eye drops solution has to be sterilized by filtration at 0.2 µm (0.2 micron), because the steam sterilization (121 °C for 15 minutes at 1·10⁵ Pa (1 atm)) destroys all components of liposomes.

To achieve such result the liposomal eyes drop solution contains liposomes built with **non** hydrogenated phospholipids containing Linseed Oil and Vitamin A Palmitate and also Vitamin E TPGS that for its specific structure improves the filterability of liposomes, but this improvement it is not enough to have a satisfactory filtration procedure, at least at industrial scale, to sterilize liposomal eyes drops. Such drawback is due to Pycnogenols presence in water phase.

To achieve such result the liposomal eyes drops solution according to the invention contains a specific and peculiar system composed of 2-Amino-2(hydroxymethyl)pro-pane-1,3-diol, that acts as salt forming agent for Pycnogenols (sparkly water soluble), and borate buffer.

Surprisingly, said liposomal eyes drops solution exerts a shield effect against UVA UVB rays (UVA 315-400 nm - UVB 315 -280). Such unexpected property is linked primarily to liposomes (plus Vitamin E TPGS) and secondarily to Vitamin B12 and Pycnogenols in water phase.

No document of prior art, such as MARTA VICARIO-DE-LA-TORRE ET AL in "Novel Nano-Liposome Formulation for Dry Eyes with Components Similar to the Preocular Tear Film", POLYMERS, vol. 10, no. 4, 11.04.2019, page 425, and CN 1850054 A, relating to "Vitamin A liposome artificial lacrimal eye drops", disclose an eye drops solution for use in the treatment of dry eye syndrome, wherein said solution comprises liposomes built with non hydrogenated phospholipids containing Linseed oil, Vitamin A Palmitate, Vitamine E TPGS , and in water phase, Vitamin B12 and pycnogenols.

### Summary of the Invention

The present invention relates to ophthalmic formulations, kits and uses thereof, wherein an eye drops solution is composed of Liposomes built with non hydrogenated phospholipids containing Linseed oil, Vitamin A Palmitate, Vitamin E TPGS and in water phase Vitamin B12 and Pycnogenol.

The presence of Pycnogenols increases the antioxidant capacity in external water phase and the presence of Vitamin E TPGS increases the antioxidant effect on lipophilic phase of liposomes.

The presence of Vitamin E TPGS, inside the liposomes, combined with the presence of Pycnogenol and Vitamin B12 outside the liposomes, have a protective effect (shield) against UV A/UVB rays.

It is further object of the invention, a liposomal eyes drops solution as above described, containing a specific and peculiar system composed of 2-Amino-2(hydroxymethyl) propane-1,3-diol, that act as salt forming agent for Pycnogenol (sparkly water soluble) and borate buffer, in order to improve the filterability of liposomes and have a satisfying filtration procedure to sterilize liposomial eyes drops only by filtration at 0.2 µm, (0,2 micron) avoiding the steam sterilization that would destroy the other components and liposomes structure.

Furthermore, the presence of 2-Amino 2(hydroxymethyl) propane-1,3-diol, that acts as salt forming agent of Pycongenols, increases the protective effect of such molecules and the filterability of the solution.

### Detailed Description of the Invention

We report below the different steps to reach the final solution according to the present invention.

The qualitative composition of the starting formulation is shown below.

**Qualitative Starting Formula**

| **Raw Material** | **CAS number** |
|---|---|
| Phospholipid S80 | 8030-76-0 |
| Linseed Oil | 6217-54-5 |
| Vitamin A palmitate | 79-81-2 |
| Hyaluronic sodium salt (1.5-1.7 MDa) | - |
| Boric Acid | 10043-35-3 |
| Sodium Tetraborate Decahydrate | 1303-96-4 |
| Sodium Chloride | 7647-14-15 |
| Distilled Water | - |

**Description and specific function of each ingredient:** Phospholipids

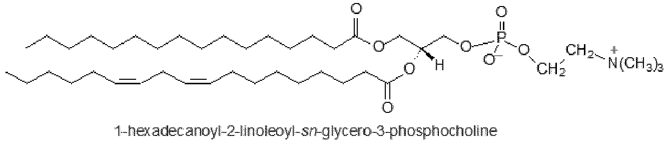

Given that the lipid layer has an important influence on the tear, understanding the structure of this portion of the tear structure is critical. This thin layer of lipid, usually 50nm to 100nm, is not a single homogeneous layer. It is believed that there is a layer of polar lipids overlying the aqueous layer of the tears, and a second layer of non-polar, hydrophobic lipids facing the air.

It is in this layer that phospholipids play an important role to stabilize the structure

Phospholipids are the peculiar constituents of liposomes.

Liposomes are composite structures made of phospholipids and may contain amounts of other molecules, such as in our case Linseed oil and Vitamin A Palmitate

Then Liposomes formed by Phospholipds (Phosphatidylcholine) exert a specific function of carriers.

In the other hand the liposomes when are on the surface of eyes deliver the molecules carried by them by a mechanism of fusion and in the meantime the phosphatidylcholine is delivered to the lipid layer that is so important for the functionality of eyes, (see Reference 3).

### Linseed Oil

The linseed oil inhibits PGE2-, leukotriene-, histamine- and bradykinin-induced inflammation. The oil also inhibits arachidonic acid-induced inflammation, suggesting its capacity to inhibit both cyclooxygenase and lipoxygenase pathways of arachidonate metabolism.

In tail immersion model, the oil raised the pain threshold to a lesser extent than morphine but showed excellent peripherally acting, analgesic activity comparable to aspirin, against acetic acid-induced writhing in mouse. In typhoid paratyphoid A/B vaccine-induced pyrexia, the oil showed antipyretic activity comparable to aspirin. The oil contains 57.38% alpha-linolenic acid.

Dual inhibition of arachidonic acid metabolism, antihistaminic and antibradykinin activities of the oil could account for the biological activity and the active principle could be alpha-linolenic acid an omega-3 (18:3, n-3) fatty acid. (Lipid modifier of eye surface with anti/inflammatory effect).

Linseed oil exerts also the function to reinforce lipophilic (oily) layer on surface of eye as that could be destroyed by dry eyes syndrome (see Reference 1)

### Vitamin A Palmitate

Retinyl Palmitate is a naturally-occurring phenyl analogue of retinol (vitamin A) with potential antineoplastic and chemopreventive activities.

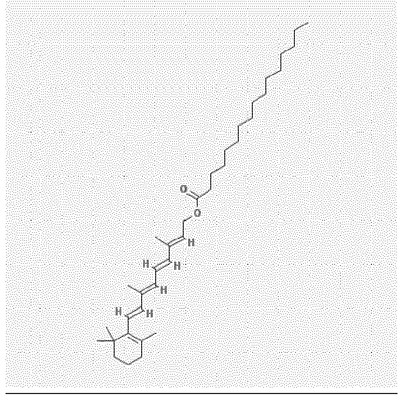

As the most common form of vitamin A taken for dietary supplementation, retinyl palmitate binds to and activates retinoid receptors, thereby inducing cell differentiation and decreasing cell proliferation. This agent also inhibits carcinogen-induced neoplastic transformation, induces apoptosis in some cancer cell types, and exhibits immunomodulatory properties.

### (NCI04)

The antioxidant activity of vitamin A and carotenoids is conferred by the hydrophobic chain of polyene units that can quench singlet oxygen, neutralize thiyl radicals and combine with and stabilize peroxyl radicals. In general, the longer the polyene chain, the greater the peroxyl radical stabilizing ability. Because of their structures, vitamin A and carotenoids can autoxidize when O2 tension increases, and thus are most effective antioxidants at low oxygen tensions that are typical of physiological levels found in tissues. (eye surface)

### Reference 2

The Vitamin A Palmitate acts as antioxidant, (0.690 V Redox potential), protecting the linseed oil, this specific action is combined to capacity to form as well as Linseed oil an lipophilic layer on eye.

### Hyaluronic Acid sodium salt

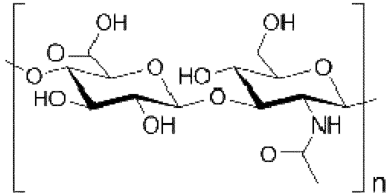

Hyaluronic acid (HA; conjugate base hyaluronate), also called hyaluronan, is an anionic, nonsulfated glycosaminoglycan distributed widely throughout connective, epithelial, and neural tissues. It is unique among glycosaminoglycans in that it is nonsulfated, forms in the plasma membrane instead of the Golgi apparatus, and can be very large, with its molecular weight often reaching the millions. It is used very often in eyes drops formulation for eye dry syndrome , (see Reference 9).

On the basis of the preceding argumentations, it is developed a quantitative starting formulation for a lot of experimental tests as follows:

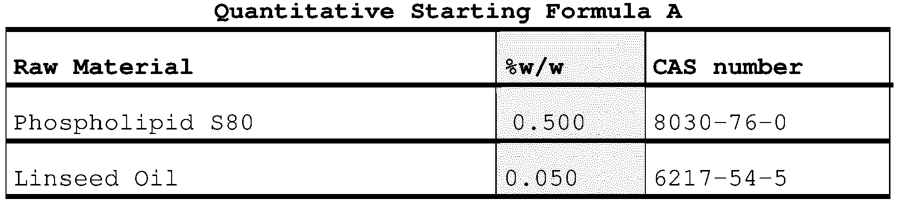

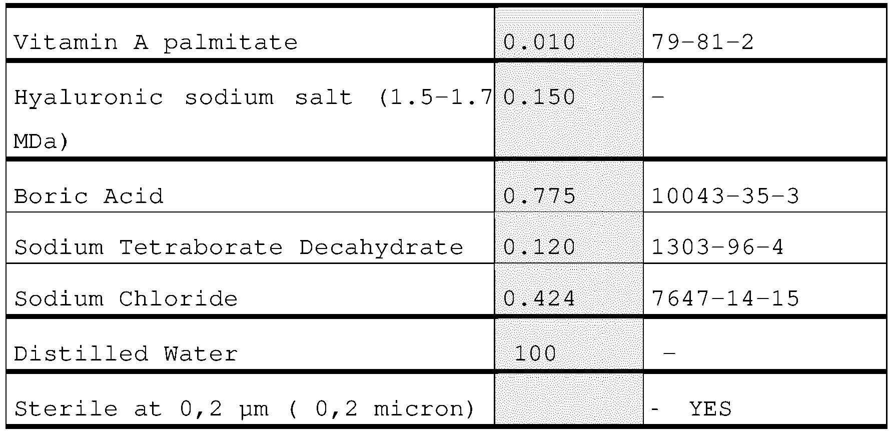

It is important to note that in order to obtain a formulation that could be a wide application on dry eyes syndrome
- the formulation has to be sterilized by filtration with a 0.2 µm (0,2 micron) filter: this procedure is compulsory because Linseed oil, Vitamin A Palmitate and Hyaluronic acid are not stable to sterilization at 121°C at 1·10⁵ Pa (1 atm) for 15 minutes.

Also the Liposomal structure composed of not hydrogenated Phosphatidylcholine is ruined by such rude treatment

From this consideration the filterability of formulation becomes a limiting factor for industrial development of Product. The preliminary work using a prototype product (5.0 litres) showed that however, even the filtration is feasible, it is very long with the possible the filter clogging, due to the presence of oil phase (Linseed Oil and Vitamin A Palmitate) inside the liposomes structure with hyaluronic acid sodium salt outside in water solution.

### Second Improvement: Test of stability

In order to overcome this technical problem, the inventor following numerous tests to increase the stability (redox capacity) and specificity of above formulation containing Linseed Oil and Vitamin A Palmitate found that the better solution was surprisingly the addition of Vitamin E.

### Vitamin E

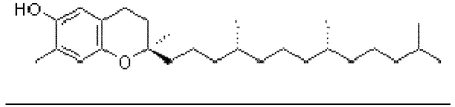

α-tocopherol/Vitamin E

In fact, Vitamin E acts as a radical scavenger, delivering an H atom to free radicals. At 323 kJ/mol, the O-H bond in tocopherols is about 10% weaker than in most other phenols.

This weak bond allows the vitamin E to donate a hydrogen atom to the peroxyl radical and other free radicals, minimizing their damaging effect.

The thus generated tocopheryl radical is relatively unreactive but revert to tocopherol by a redox reaction with a hydrogen donor such as vitamin C.

As it is fat-soluble, it is incorporated into cell membranes, which are protected from oxidative damage by vitamin E.

Thus, α-Tocopherol Vitamin E protects Vitamin A in eyes.

In other words, interactions between alpha-tocopherol (vitamin E) and all-trans retinol (Vitamin A) suppress lipid peroxidation using unilamellar liposomal system of phosphatidylcholine from either egg or soybean. (see Reference 6)

Taking into account this result, the Applicant changed the formula as follows:

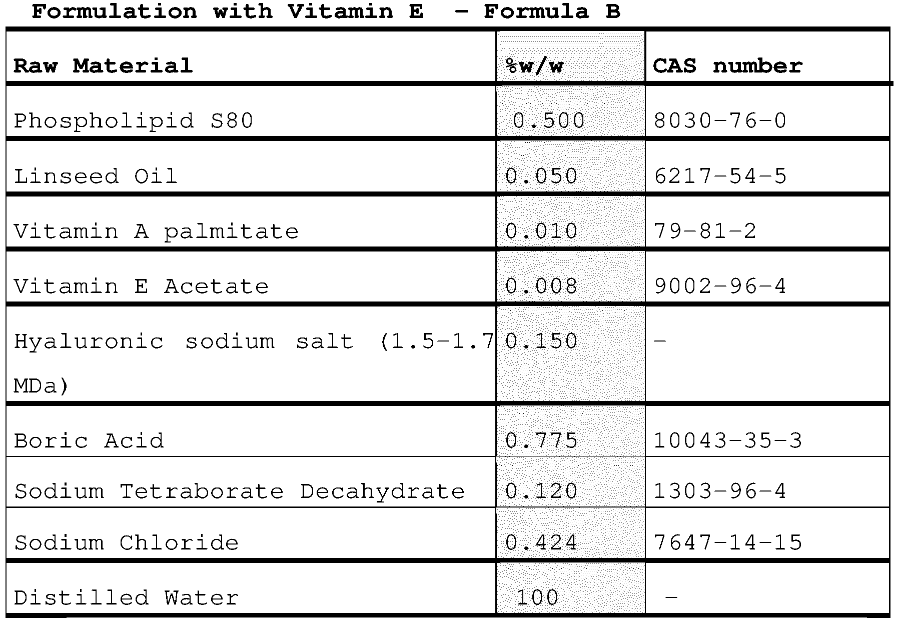

The preliminary work using a prototype product (5 litres) of formula B (formulation with Vitamin E) showed that the filtration of such formula is enough easy, even if few difficulties occur during the filtration process, due to the presence of oil phase (Linseed Oil, Vitamin A Palmitate and Vitamin E Acetate) inside the liposomes structure with hyaluronic acid sodium salt outside the liposomes.

### Third improvement

To increase the stability and specificity of above formulation B containing Linseed Oil, Vitamin A Palimitate and Vitamin E Acetate we added to previous formulation the Pycnogenols extract assuming that should have antioxidant effect.

The Pycnogenols extract should exert, also, a shield effect against UV rays insult on ocular surface.

### Pycnogenols extract

It is combination of bioflavonoids from pine bark of French maritime pine and scavenges free radicals, with an anti inflammatory effects and promotes cellular health exerts is antioxidant effect in hydrophilic milieu.

Between 65% and 75% of Pycnogenol are procyanidins comprising of catechin and epicatechin subunits with varying chain lengths. Other constituents are polyphenolic monomers, phenolic or cinnamic acids and their glycosides. As many studies indicate, pycnogenol components are highly bioavailable.

Uniquely, pycnogenol displays greater biological effects as a mixture than its purified components do individually indicating that the components interact synergistically.

Pycnogenol can protect the chain of lipid bilayer against oxidative damage to fatty acids.

During the process of lipoperoxidation, free radicals react with polyunsaturated fatty acids first resulting in the formation of conjugated dienes from which peroxyl radicals and hydrolipoperoxides are formed in aerobic conditions.

These products decrease the production of thiobarbituric acid-reactive substances (TBARS), indicating that the protective effect of PYC is due to its antioxidant activity.

From the tests result that the antioxidant action of Pycnogenol in the proposed formulation B has a dual goals:
protect eye surface from free radicals even in hydrophilic compartment,
protect the no Hydrogetated phospholipids that are the building stones of liposomes and are carriers of Linseed Oil and Vitamin A Palmitate and Vitamin E Acetate. (see Reference 4)

Thus, we defined a new formulation with Pycnogenols extract as follows:

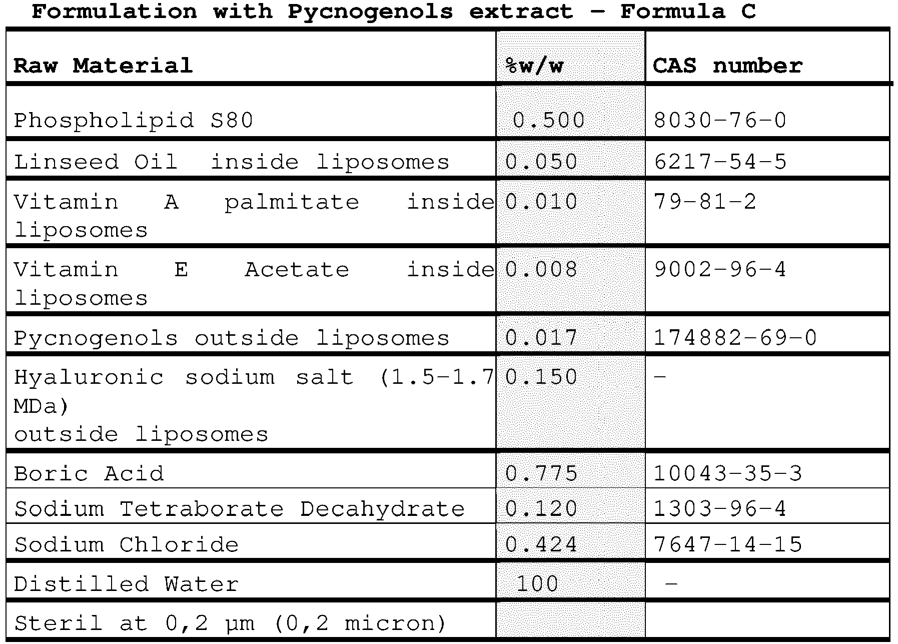

However the filtration test using a prototype product(5 liters of Formula C) showed that the Liposomes formulation in presence of Pycnogenols becomes not easily filterable at 0.2 micron.

Moreover Picnogenols, before and after sterilization at 0.2 micron, do not appear perfectly solubilized and their color is Reddish;

Furthermore the final product C looks with not well defined color with some "micro fines" dispersed on solution outside the liposomes.

This means for the formulation C several critical points has to be solved like:
- not clear, not limpid appearance
- product stability/ sedimentation,
- a poor shield effect of Pycnogenols against UVA/UVB rays because appear not completely soluble.

In view of said unsatisfactory results, several formulations, below reported, have been prepared to overcome or at least to reduce, the critical points above indicated.

**Improvement of Appearance:** To mask the not well defined color of formulation C we added Vitamin B12 that have in water solution a strong Red Color.

### Vitamin B12

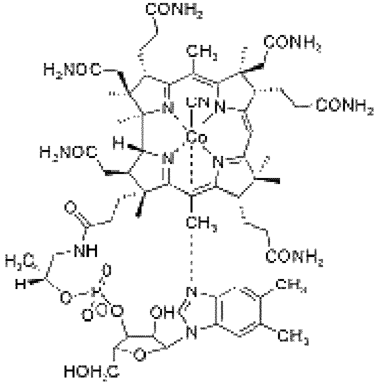

It is well known that B12 deficiency is related with Neuropatic ocular pain, this pharmacological action is linked to chemical physical peculiarity of such Vitamin.

The presence of Vitamin B12, that have a clear red color when solubilized in water, gives to the final product D a well defined Pink color, improving the visual appearance of the product. (see Reference 5)

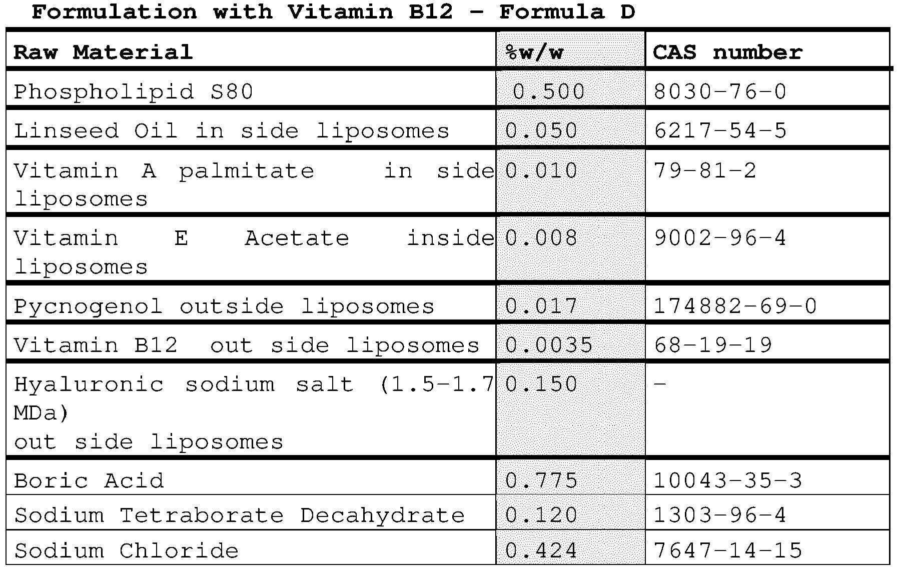

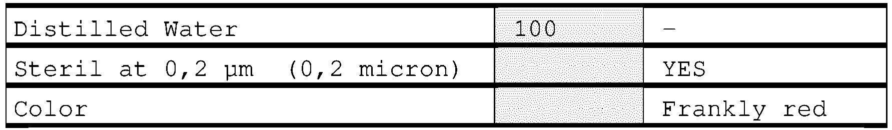

The preliminary work using a prototype product (5 liters) showed that the filtration of such formula D is very difficult, due the presence of oil phase (Linseed Oil, Vitamin A Palmitate and Vitamin E Acetate) inside the liposomes structure and hyaluronic acid sodium salt and Pycnogenols and Vitamin B12 outside in water phase.

Instead, the appearance of final product is very good, its color is PINK, the presence of Pycnogenols ( microfines) masked by the strong PINK COLOR of Vitamin B12.

However, the formulation Critical Concerns:
1) Possible instability of formulation (sedimentation of Pycnogenols microfines joined to not efficient shield effect against UV rays)
2) The difficulty to filtrate the formulation remain unsolved.

### First Attempt to Improve Filtration Performance

To increase the filtration performance we substitute in above formulation Vitamin E acetate with VITAMIN E TPGS, this derivative of Vitamin E should improve the filterability of all composition saving antioxidant capacity, at least in water phase , by increasing the solubility of Pyncnogenols extract

### Vitamin E TPGS

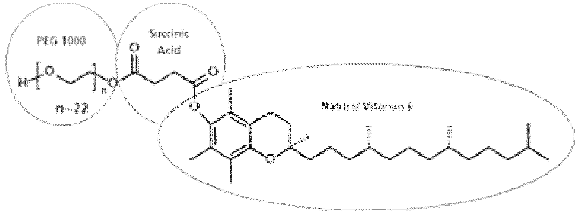

D-α-tocopheryl polyethylene glycol succinate (Vitamin E TPGS, or simply TPGS) is a water-soluble derivative of natural Vitamin E, which is formed by esterification of Vitamin E succinate with polyethylene glycol (PEG). As such, it has advantages of PEG and Vitamin E in application of various nanocarriers for drug delivery, including extending the half-life of the drug in plasma and enhancing the cellular uptake of the drug.

TPGS has an amphiphilic structure of lipophilic alkyl tail and hydrophilic polar head with a hydrophilic/lipophilic balance (HLB) value of 13.2 and a relatively low critical micelle concentration (CMC) of 0.02% w/w, which make it to be an ideal molecular biomaterial in developing various drug delivery systems, including pro drugs, micelles, liposomes and nanoparticles, which would be able to realize sustained, controlled and targeted drug delivery as well as to overcome multidrug resistance (MDR) and to promote oral drug delivery as an inhibitor of P-glycoprotein (P-gp) (see Reference 7)

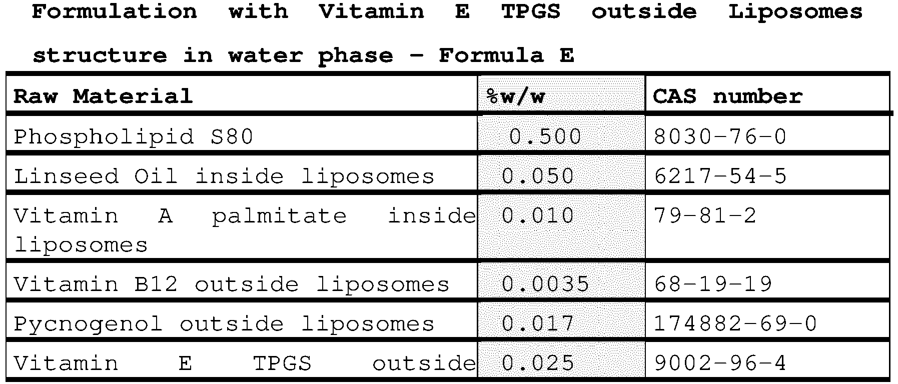

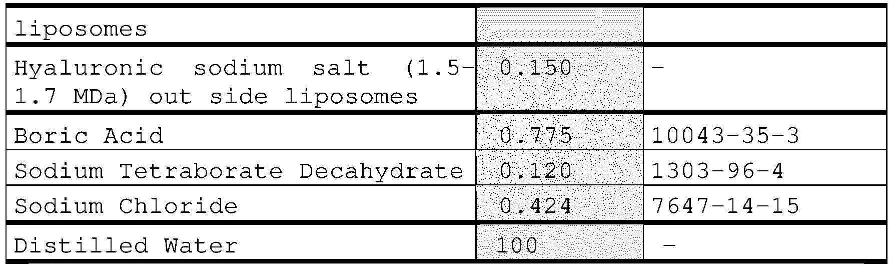

The preliminary work using such prototype (5 liters), containing Vitamin ETPGS, outside the liposomes showed that the filtration of such formula is feasible and easier respect to formulation with Vitamin E Acetate, even if the antioxidant power inside lipophilic phase clearly is diminished.

This improved filtration performance means that Vitamin E TPGS increases the Fluidity of all mixture composed by liposomes (containing Vitamin A Palmitate, Leenseed Oil) Vitamin B12 (freely soluble in water), Hyaluronic acid sodium salt (soluble in water).

The performance of filtration is almost good, (but not easy and complete), this improvement is due the Presence of Vitamin ETPGS in solution outside the liposomes, but some concerns on Pycnogenols still remain.

In order to improve the filterability of the liposomial solution, and maintain the antioxidant power on lipophilic phase, we added Vitamin E ETPGS inside the liposomal structure, obtaining surprisingly a further improvement of the filterability.

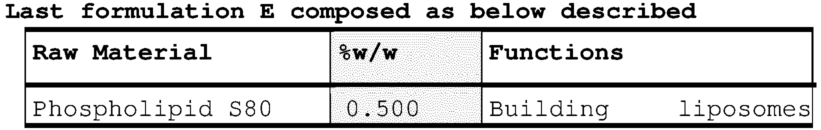

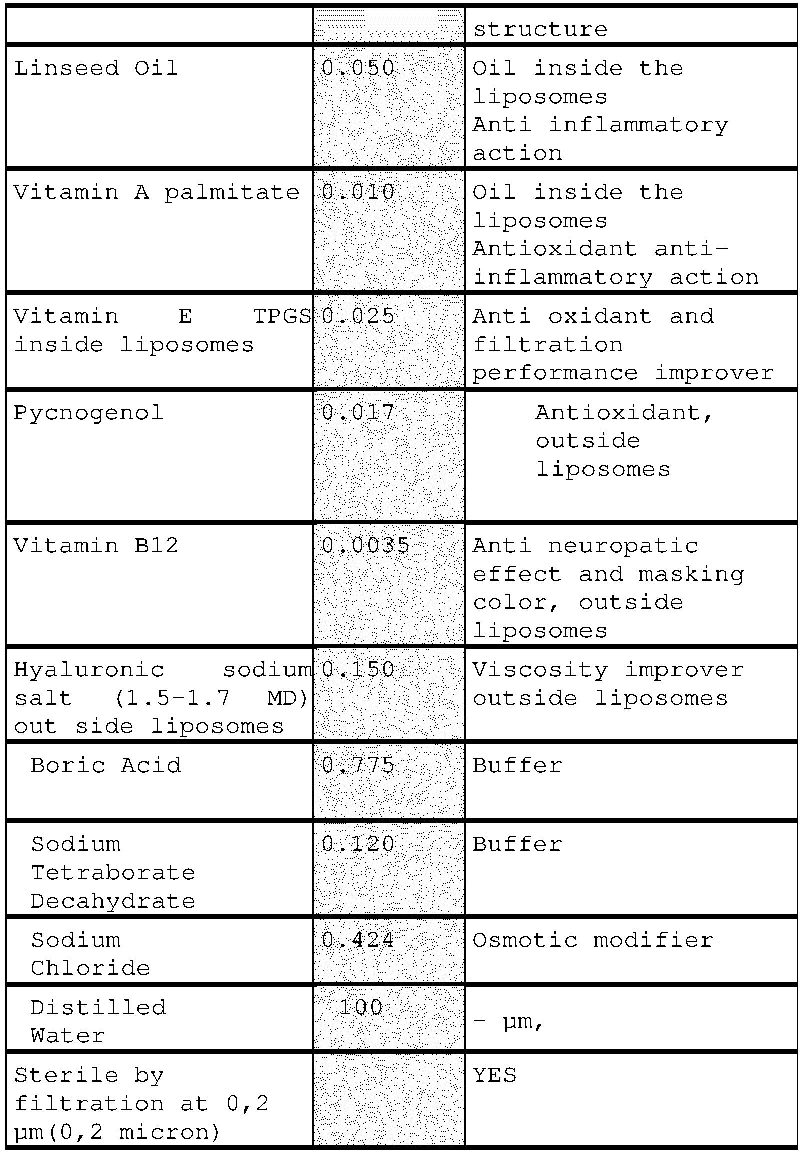

This improved filtration performance means that Vitamin E TPGS increases the fluidity of liposomes (containing Vitamin A Palmitate, Leenseed Oil), such increased liposomal fluidity exerts a positive effect on filterability of all mixture composed liposomes and Vitamin B12 (freely soluble in water), Hyaluronic acid sodium salt (soluble in water) and Pycnogenols that still remain not perfectly filterable

In few words this improvement is good but not enough to guaranty an easy and reliable sterilization by filtration at 0.2 µm, (0,2 micron) at least at industrial scale, 100-200 litres each time.

This difficulty pushed us to focus our attention on peculiar unexpected behavior of Pycnogenols extract.

In fact Pycnogenol is declared soluble in water, but the difficulties that we had on filtration at 0.2 µm (0,2micron)indicate that this statement is not really true.

Pycnogenol^{®} contains the bioflavonoids as catechin, epicatechin and taxifolin as well as phenolcarbonic acids, such molecules are very weak acid and are slightly soluble in water

We report below the chemical structures of cathechin, epicatechin and taxifolin:

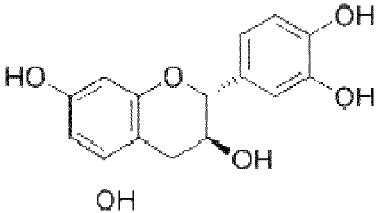

### Catechin

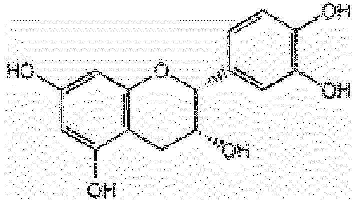

### (-)-Epicatechin (2R,3R)

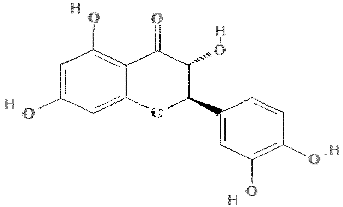

### Taxifolin

The chemical structures above reported, referring at least to part of components of Pycnogenols mixture, indicate that such molecules, to be freely soluble in water have to be modified in Salts Forms.

To clarify this points we prepared the following solutions:
Pycnogenols 0.034 % in Water;
Pycnogenols 0.034 % in Decahydrate Borate solution (final pH 7.05); 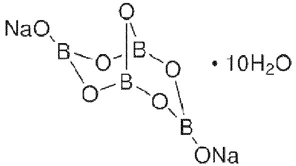 SODIUM BORATE, DECAHYDRATE ,
   and
Pycnogenols 0.034 % in Tris solution (final pH 7.1). 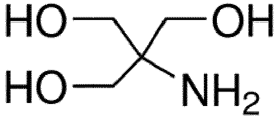 Tris (2-Amino-2-(hydroxymethyl)propane-1,3-diol)
Pynogenol Tris buffer solution is RED.
Pynogenol Borate buffer solution is REDDISH.

### Second Attempt to Improve Filtration Performance

To increase the filtration performance we added in above formulation Tris ((2-Amino-2(hydroxymethyl) propane-1,3-diol), to increase the solubility of Pyncnogenols extract.

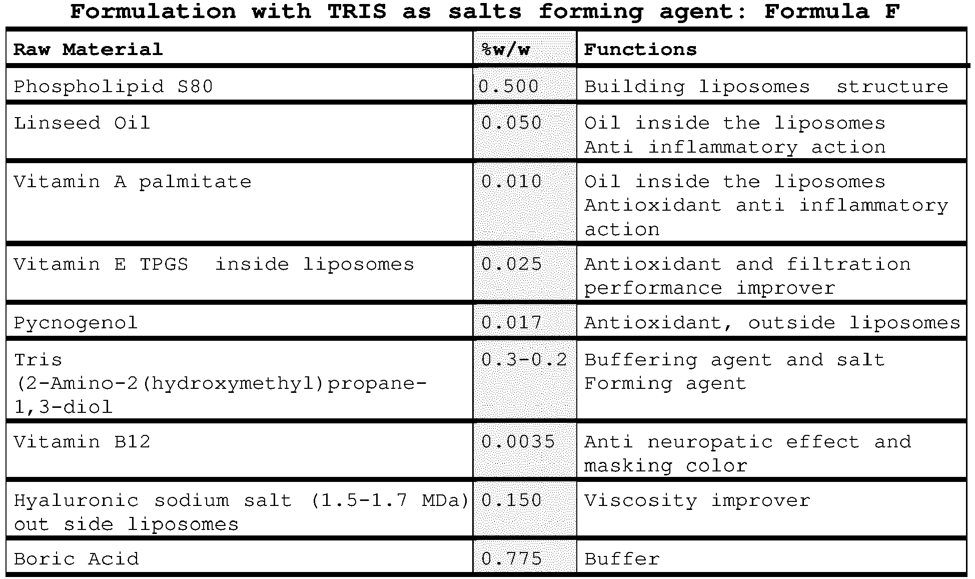

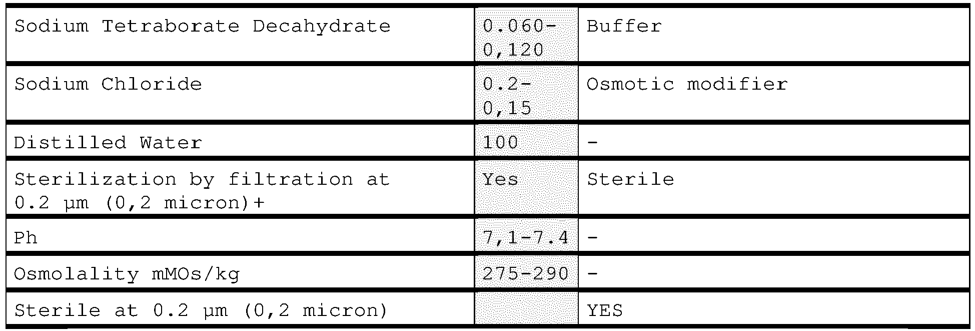

Last formulation composed,(formula F) as above described, can be sterilized by filtration with 0.2 µm (0,2 micron) filter: the performance of filtration is Excellent .

The improvement is due to the presence of Tris(2-Amino-2(hydroxymethyl)propane-1,3-diol) that reacting with Phenolic groups of catechin like molecules, produce salts (an ion pair) very soluble in water, (see References 10/11).

This improved filtration performance is then due to the combined effect of Vitamin E TPGS inside of liposomes, and Tris outside of liposomes.

### Conclusions

The activity performed allow us to produce a Formulation, (Formula F) that could be a wide application on dry eyes syndrome because it contains components that:
have anti inflammatory action,
have antioxidant action,
Counteract Neuropatic Ocular Pain Restore the oil layer on eye surface,
Maintain moisture.

The presence of Pycnogenols increases the antioxidant capacity in external water phase and the presence of Vitamin E TPGS increases the antioxidant effect on lipophilic phase of liposomes

The activity performed allow us to produce a Formulation at industrial scale, with excellent filterability, that results are linked to specific presence of Vitamin E TPGS and addition of TRIS that exerts a peculiar function of solubilizing, (salt forming) Pycnogenols

### Further test: Protective effect against UVA/UVB rays

The presence of Vitamin E TPGS, inside the liposomes, combined with presence of Pycnogenols and Vitamin B12, outside the liposomes, should have a Protective (shield) effect against UVA/UVB rays.

To evaluate such hypothesis, we irradiated for 15 minutes with UVA/UVB Lamp* several preparations, listed below containing Vitamin A Palmitate:
- Vitamin A Palmitate 0.010% Solution;
- Vitamin A Palmitate 0.010%, Vitamin E TPGS 0.025% inside Liposomes;
- Vitamin A Palmitate 0.010%, Vitamin E TPGS 0.025% in Liposomes plus Pycnogenols 0.017% in water phase;
- Vitamina A Palmitate 0.010%, Vitamin E TPGS 0.025% inside Liposomes plus Vitamin B12 0.0035% in water phase; and
- Vitamin A Palmitate 0.010% + Vitamina E TPGS 0.025% inside Liposomes plus Pycnogenol 0.017% + Vitamin B12 0.0035% in water phase.

We measured the concentration of Vitamin A Palmitate before and after this treatment, the table 1 below summarizes the results obtained.

**TABLE 1**

| | Vitamin A Palmitate % before UVA/UVB treatment | Vitamin A Palmitate % after UVA/UVB treatment | Degradation % |
|---|---|---|---|
| Vitamin A Palmitate 0.010% Solution | 0.0100 % | 0.0020 % | 80 |
| Vitamin A Palmitate 0.010% Vitamin ETPGS0.025% inside Liposomes | 0.0100 % | 0.0040 % | 60 |
| Vitamin A Palmitate 0.010% Vitamin ETPGS 0.025% in Liposomes plus Pycnogenols 0.017% in water phase | 0.0100 % | 0.0050% | 50 |
| Vitamina A Palmitate 0.010% VitaminETPGS0.025% inside Liposomes plus Vitamin B12 0.0035% in water phase | 0.0100 % | 0.0045 % | 55 |
| Vitamin A Palmitate 0.010% + Vitamina E TPGS 0.025% inside Liposomes plus Pycnogenol 0.017% + Vitamin B12 0.0035% in water phase | 0.0100 % | 0.0065 % | 35 |

| | | | |
|---|---|---|---|
| *Irradiation power (intensity at different wave lengths)), see Reference (12). • 200 nm 60mWatt • 250 nm 90 mWatt • 300 nm 125 mWatt • 350 nm 137 mWatt • 400 nm 118 mWatt | | | |

Such results demonstrate a shield effect against UVA/UVB insult.

Thus the Vitamin A Palmitate is protected by:
- Liposomes containing Vitamin E TPGS as such the Vitamin A Palmitate is protected by:
- Vitamin ETPGS inside the liposomes structure, and
- Pycnogenols, Vitamin B12 outside the liposomes, inside the water phase

The evaluation of degradation of Vitamin A Palmitate is only simple (trivial) tool to highlight such protection, in the other hand this shield effect can be exerted to ocular surface (Reference 12) with evident advantage for patients suffering of dry eye syndrome or people that have to work for long time with light emitting device like computer screen

### Final conclusions

The activity, above described, explains how to produce, at industrial scale, an eye drops solution composed of liposomes built with not hydrogenated phospholipids containing Linseed oil, Vitamin A Palmitate, Vitamin E TPGS, and in water phase Vitamin B12 and Pycnogenols.

This liposomal eye drops solution has to be sterilized by filtration at 0.2 µm, because the steam sterilization (121 °C for 15 minutes at 1·10⁵ Pa (1 atm) destroys all components of liposomes.

To achieve such result the liposomal eyes drop solution contains liposomes built with not hydrogenated phospholipids containing Vitamin E TPGS that for its specific structure improves the filterability of liposomes, but this improvement it is not enough to have a satisfying filtration procedure to sterilize liposomal eyes drops.

To achieve such result the liposomal eyes drops solution contains a specific and peculiar system composed of 2-Amino-2(hydroxymethyl)pro-pane-1,3-diol, that acts as salt forming agent for Pycnogenols (sparkly water soluble), and borate buffer.

The liposomal eyes drops exerts a shield effect against UVA UVB rays (UVA 315-400 nm - UVB 315-280), such unexpected property is linked primarily to liposomes (plus Vitamin E TPGS) and secondarily to Vitamin B12 and Pycnogenols in water phase.

### Process for preparation of the Eyes Drop Solution

According to another aspect of the invention, the Applicant has developed a process for the preparation of liposomial eye drops solution for use in the treatment of Dry eye syndrome comprising the following three steps:

### First step:

### Preparation of Liposomes carrying Lipophilic substances

1) Solubilize all lipophilic ingredients, Not Hydrogenated Phospholipids, Linseed oil, Vitamin A Palmitate plus Vitamin E TPGS vitamin by a solvent usually Ethanol;
2) The solvent is removed under vacuum to obtain a dry powder;
3) The powder is dispersed in borate buffer and mixed by a mechanical mixer, a solution of large liposomes is obtained, that could be defined as Pro liposomes;
4) The solution of such large liposomes is then extruded at high pressure, in a range of 600/1000 bar;
5) The extrusion procedure at High pressure is repeated several times *4/7* to obtain liposomes of size less than 200 nm.

In a preferred embodiment 100 liters of Liposomes Solution is prepared with the following Formula:

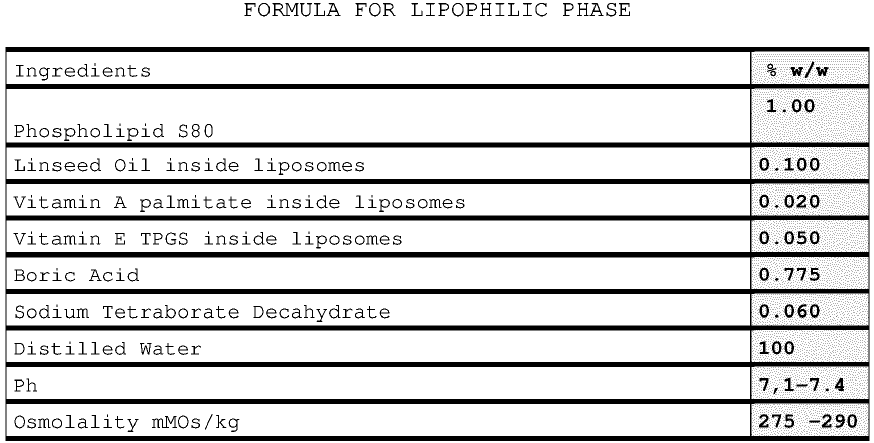

### Second step:

### Preparation of Water Solution containing Hydrophilic Substances

6) Solubilize in distilled water Pycnogenols, Tris, Vitamin B12, Hyaluronic Acid and Borate buffer;
7) A clear and red colored solution is obtained.

In a preferred embodiment 100 liters of Water solution is prepared with the following Formula:

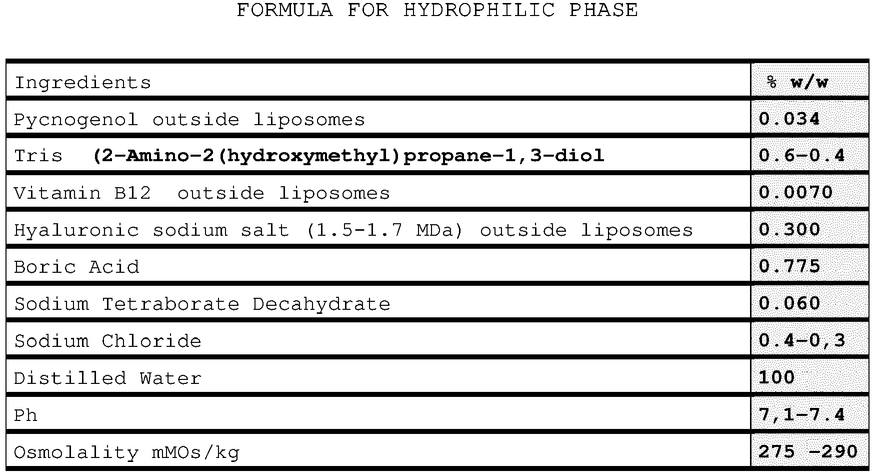

### Third step:

**Preparation of Final Sterile Eyes Drops Solution** 8) Liposomes solution (100 liters), prepared at First step, is added to water colored Solution (100 liters), prepared at second step, obtaining a Final Standard Eyes Drop Solution, sterilized by filtration at 0.2 µm (0,2 micron) filter, with the following formula:

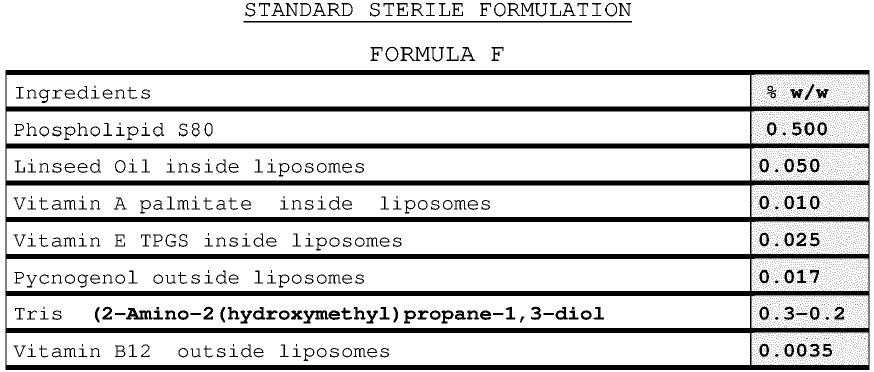

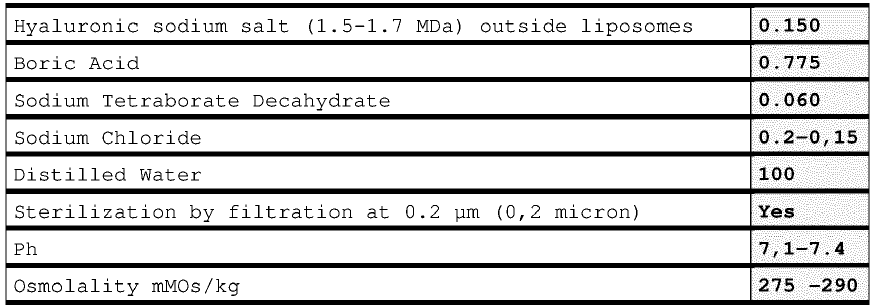

In a preferred embodiment 200 liters of Standard EYES Drop Solution is prepared with the above Standard Formula F.

Modification of the above described modes for carrying out the invention that are obvious to person of skill in the art to which the invention pertains are intended to be within the field of the invention as defined by the following claims.

### BIBLIOGRAPHY

**1)** Anti-inflammatory, analgesic and antipyretic activities of Linum usitatissimum L. (flaxseed/linseed) fixed oil. Kaithwas G1, Mukherjee A, Chaurasia AK, Majumdar DK.
2) Ocul Surf. 2009 Oct;7(4):176-85. Antioxidant defenses in the ocular surface. Chen Y1, Mehta G, Vasiliou V.
3) Are Phospholipids the Critical Ingredient?. Donald Korb, O.D., and Ralph Stone, Ph.D.
4)The effects of pycnogenol on antioxidant enzymes in a mouse model of ozone exposure Min-Sung Lee,1 Kuk-Young Moon,1 Da-Jeong Bae,1 Moo-Kyun Park,2 and An-Soo Jang 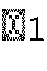
5) Vitamin B12 deficiency evaluation and treatment in severe dry eye disease with neuropathic ocular pain. Graefes Arch Clin Exp Ophthalmol. 2017 Jun;255(6):1173-1177. doi: 10.1007/s00417-017-3632-y. Epub 2017 Mar 15. Ozen S1, Ozer MA2, Akdemir MO³.
6)Arch Biochem Biophys. 1996 Feb 1;326(1):57-63. Synergistic interactions between vitamin A and vitamin E against lipid peroxidation in phosphatidylcholine liposomes. Tesoriere L1, Bongiorno A, Pintaudi AM, D'Anna R, D'Arpa D, Livrea MA.
7) Vitamin E TPGS as a Molecular Biomaterial for Drug Delivery Biomaterials 33(19):4889-906·April 2012 with 203 Reads DOI: 10.1016/j.biomaterials.2012.03.046 Source: PubMed
8) Using Tocophersolan for Drug Delivery:A natural vitamin E derivative is an innovative excipient Jan 02, 2015 by Yves Robin Pharmaceutical Technology Volume 39, Issue 1
9)Pucker AD, Ng SM, Nichols JJ (2016). "Over the counter (OTC) artificial tear drops for dry eye syndrome". Cochrane Database Syst Rev.2: CD009729.
10) UltraPure^{™} Buffer-Saturated Phenol Catalog number: 15513047
11) Ketorolac Trometamin salt anti inflammatory drug 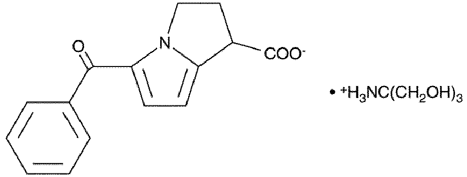 ID CAS: 74103-06-3 Molar mass: 255,27 g/mol
12) VIII. Évfolyam 3. szam - 2013. szeptember Kolonics Gabor THE EXAMINATION OF UV ABSORPTION OF POLYPHENOLS (NATURAL SUBSTANCE)

## Claims

1. An eye drops solution for use in the treatment of Dry eye syndrome **characterized in that** it comprises Liposomes built with non hydrogenated phospholipids containing Linseed oil, Vitamin A Palmitate, Vitamin E TPGS, and in water phase, Vitamin B12 and Pycnogenols.

2. The eye drops solution as in claim 1 **characterized in that** the Liposomal Eyes Drops solution contains furthermore a system composed of 2-Amino-2(hydroxymethyl)pro-pane-1,3-diol, that acts as salt forming agent of Pycnogenols, sparkly water soluble, and buffer pH regulator.

3. The eye drops solution according to each of preceding claims, **characterized in that** it is sterilized by filtration at 0.2 µm, because the steam sterilization achieved by exposing said solution to saturated steam at high temperatures at 121 °C for 15 minutes at 1·10⁵ Pa (1 Atm), destroys all components of liposomes.

4. The eye drops solution according to each of preceding claims, **characterized in that** the liposomal eyes drops exert a shield effect against UVA UVB rays (UVA 315-400 nm - UVB 315 -280), such property being linked primarily to liposomes (plus Vitamin E TPGS) and secondarily to Vitamin B12 and Pycnogenols in water phase.

5. The eye drops solution according to each of claims 2-4, **characterized in that** it has the following composition:

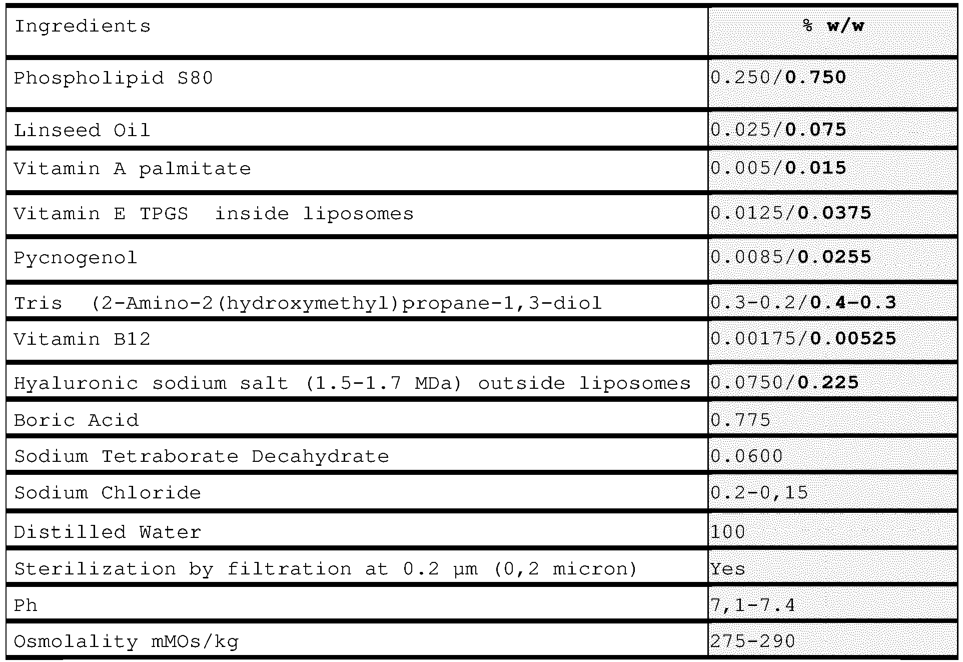

6. The eye drops solution according to claim 5, wherein the composition is as follows:

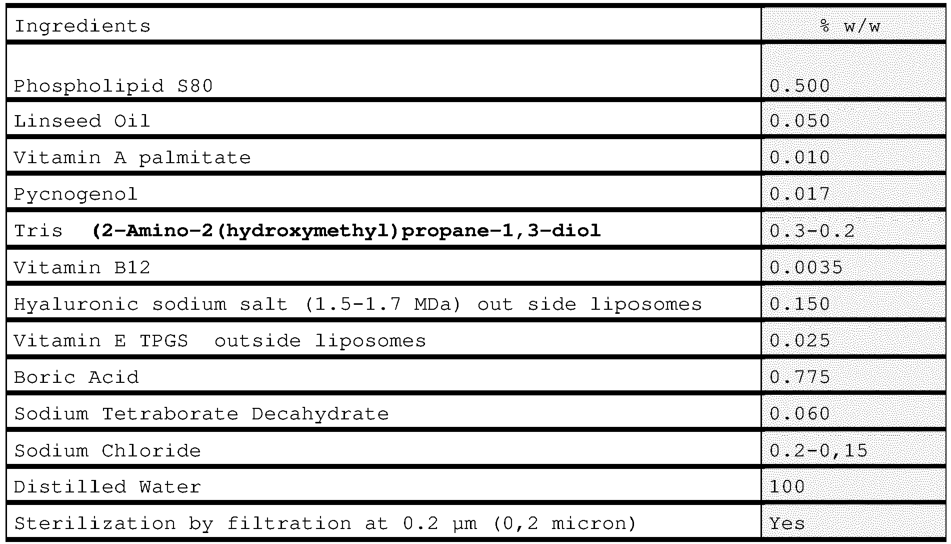

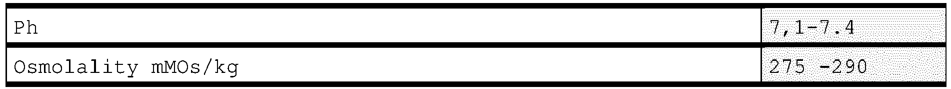

7. Process for preparation of an eye drops solution for use in the treatment of dry eye syndrome, comprising the following steps:
First step:
Preparation of Liposomes carrying Lipophilic substances:
- Solubilize all lipophilic ingredients, not hydrogenated phospholipids, linseed oil, Vitamin A Palmitate plus Vitamin E TPGS by a solvent usually Ethanol,
- The solvent is removed under vacuum to obtain a dry powder,
- The powder is dispersed in borate buffer and mixed by a mechanical mixer, a solution of large; liposomes is obtained, that could be defined as Pro liposomes;
- The solution of such large liposomes is then extruded at high pressure, between 600/1000 bar;
- The extrusion procedure at High pressure is repeated several times, between 4 and 7, to obtain liposomes of size less than 200 nm;
Second step:
Preparation of Water Solution containing
Hydrophilic Substances
- Solubilize in distilled water Pycnogenols, Tris, Vitamin B12, Hyaluronic Acid and Borate buffer;
wherein the concentration of Lipophilic substances in the first step and the concentration of Hydrophilic substances in the second step are such as to permit, added each other in volume 1:1, to get the solution with the desired concentration;
- A clear and red colored solution is obtained;
Third step:
Preparation of Final Sterile Eyes Drops:
An amount "X" of liposomes solution, prepared at first step, is added to a same amount "X" of water colored solution, prepared at second step, obtaining an amount of "2X" of a final standard eyes drop solution of the formula according to claim 5, and
the final standard eye drop solution is sterilized by filtration at 0.2 µm (0,2 micron) filter.

8. The eye drops solution according to each of claims 4 and 5 for use in the treatment of Dry eye syndrome **characterized in that** it is used as shield against UVA/UVB rays.

9. The eye drops solution according to each of the preceding claims 1-6 for use in the treatment of Dry eye syndrome **characterized in that** it is to reduce the negative effects of dry eyes syndrome.

10. The eye drops solution obtained by the process according to claim 7 for use in the treatment of Dry eye syndrome **characterized in that** it is used as shield against UVA/UVE rays.

## Patentansprüche

1. Augentropfenlösung zur Verwendung bei der Behandlung des Trockenes-Auge-Syndroms, **dadurch gekennzeichnet, dass** sie Liposome umfasst, die aus nicht-hydrierten Phospholipiden aufgebaut sind, die Leinsamenöl, Vitamin-A-Palmitat, Vitamin-E-TPGS und, in der Wasserphase, Vitamin B12 und Pycnogenole enthält.

2. Augentropfenlösung wie in Anspruch 1, **dadurch gekennzeichnet, dass** die liposomale Augentropfenlösung ferner ein System enthält, das aus 2-Amino-2(hydroxymethyl)propan-1,3-diol, das als salzbildendes Mittel von Pycnogenolen agiert und sprudelwasserlöslich ist, und einem Puffer-pH-Regulator besteht.

3. Augentropfenlösung nach jedem der vorhergehenden Ansprüche, **dadurch gekennzeichnet, dass** sie durch Filtration mit 0,2 µm sterilisiert wird, weil die Dampfsterilisation, die erreicht wird, indem die Lösung für 15 Minuten gesättigtem Dampf bei hohen Temperaturen von 121 °C bei 1·10⁵ Pa (1 Atm) ausgesetzt wird, alle Komponenten von Liposomen zerstört.

4. Augentropfenlösung nach jedem der vorhergehenden Ansprüche, **dadurch gekennzeichnet, dass** die liposomalen Augentropfen einen Abschirmeffekt gegen UVA/UVB-Strahlen (UVA 315-400 nm - UVB 315-280 nm) haben, wobei eine derartige Eigenschaft primär mit Liposomen (plus Vitamin-E-TPGS) und sekundär mit Vitamin B12 und Pycnogenolen in der Wasserphase verknüpft wird.

5. Augentropfenlösung nach jedem der Ansprüche 2-4, **dadurch gekennzeichnet, dass** sie die folgende Zusammensetzung aufweist:
| Inhaltsstoffe | Gew.-% |
|---|---|
| Phospholipid S80 | 0,250/**0,750** |
| Leinsamenöl | 0,025/**0,075** |
| Vitamin-A-Palmitat | 0,005/**0,015** |
| Vitamin-E-TPGS innerhalb von Liposomen | 0,0125/**0,0375** |
| Pycnogenol | 0,0085/**0,0255** |
| Tris(2-Amino-2(hydroxymethyl)propan-1,3-diol | 0,3-0,2/**0,4-0,3** |
| Vitamin B12 | 0,00175/**0,00525** |
| Hyaluron-Natriumsalz (1,5-1,7 MDa) außerhalb von Liposomen | 0,0750/0,225 |
| Borsäure | 0,775 |
| Natriumtetraborat-Decahydrat | 0,0600 |
| Natriumchlorid | 0,2-0,15 |
| Destilliertes Wasser | 100 |
| Sterilisation durch Filtration mit 0,2 µm (0,2 Mikronen) | Ja |
| Ph | 7,1-7,4 |
| Osmolalität mMOs/kg | 275-290 |

6. Augentropfenlösung nach Anspruch 5, wobei die Zusammensetzung wie Folgt ist:
| Inhaltsstoffe | Gew.-% |
|---|---|
| Phospholipid S80 | 0,500 |
| Leinsamenöl | 0,050 |
| Vitamin-A-Palmitat | 0.010 |
| Pycnogenol | 0,017 |
| Tris(2-Amino-2(hydroxymethyl)propan-1,3-diol | 0,3-0,2 |
| Vitamin B12 | 0,0035 |
| Hyaluron-Natriumsalz (1,5-1,7 MDa) außerhalb von Liposomen | 0,150 |
| Vitamin-E-TPGS außerhalb von Liposomen | 0,025 |
| Borsäure | 0,775 |
| Natriumtetraborat-Decahydrat | 0,060 |
| Natriumchlorid | 0,2-0,15 |
| Destilliertes Wasser | 100 |
| Sterilisation durch Filtration mit 0,2 µm (0,2 Mikronen) | Ja |
| Ph | 7,1-7,4 |
| Osmolalität mMOs/kg | 275-290 |

7. Prozess zur Herstellung einer Augentropfenlösung zur Verwendung bei der Behandlung des Trockenes-Auge-Syndroms, welcher die folgenden Schritte umfasst:
Erster Schritt:
Herstellung von Liposomen, die lipophile Substanzen tragen:
- Solubilisieren aller lipophilen Inhaltsstoffe, nicht-hydrierten Phospholipide, Leinsamenöl, Vitamin-A-Palmitat plus Vitamin-E-TPGS durch ein Lösemittel, üblicherweise Ethanol;
- Das Lösemittel wird unter Vakuum entfernt, um ein trockenes Pulver zu erhalten;
- Das Pulver wird in Borat-Puffer dispergiert und durch einen mechanischen Mischer gemischt, es wird eine Lösung aus großen Liposomen erhalten, die als Pro-Liposome definiert werden können;
- Die Lösung aus derartigen großen Liposomen wird dann bei hohem Druck zwischen 600/1000 bar extrudiert;
- Der Extrusionsvorgang bei hohem Druck wird mehrere Male, zwischen 4 und 7 Mal, wiederholt, um Liposome mit einer Größe von weniger als 200 nm zu erhalten;
Zweiter Schritt:
Herstellung einer Wasserlösung, die hydrophile Substanzen enthält:
- Solubilisieren der Pycnogenole, Tris, Vitamin B12, Hyaluronsäure und Borat-Puffer in destilliertem Wasser;
wobei die Konzentration lipophiler Substanzen im ersten Schritt und die Konzentration hydrophiler Substanzen im zweiten Schritt derart sind, dass gestattet wird, dass, einander zugegeben in einem Volumen von 1:1, die Lösung mit der gewünschten Konzentration erhalten wird;
- Es wird eine klare Lösung mit roter Farbe erhalten;
Dritter Schritt:
Herstellung der abschließenden sterilen Augentropfen:
Eine Menge "X" der Liposomenlösung, hergestellt im ersten Schritt, wird zu einer gleichen Menge "X" der farbigen Wasserlösung, hergestellt im zweiten Schritt, zugegeben, wodurch eine Menge "2X" einer abschließenden Standard-Augentropfenlösung mit der Formel nach Anspruch 5 erhalten wird, und
die abschließende Standard-Augentropfenlösung wird durch Filtration mit einem 0,2 µm (0,2 Mikronen) Filter sterilisiert.

8. Augentropfenlösung nach jedem der Ansprüche 4 und 5 zur Verwendung bei der Behandlung des Trockenes-Auge-Syndroms, **dadurch gekennzeichnet, dass** sie als eine Abschirmung gegen UVA/UVB-Strahlen verwendet wird.

9. Augentropfenlösung nach jedem der vorhergehenden Ansprüche 1-6 zur Verwendung bei der Behandlung des Trockenes-Auge-Syndroms, **dadurch gekennzeichnet, dass** sie die negativen Auswirkungen des Trockenes-Auge-Syndroms verringern soll.

10. Augentropfenlösung, die durch den Prozess nach Anspruch 7 erhalten wird, zur Verwendung bei der Behandlung des Trockenes-Auge-Syndroms, **dadurch gekennzeichnet, dass** sie als eine Abschirmung gegen UVA/UVB-Strahlen verwendet wird.

## Revendications

1. Solution de gouttes pour les yeux pour une utilisation dans le traitement du syndrome des yeux secs **caractérisée en ce qu'**elle comprend des liposomes construits avec des phospholipides non hydrogénés contenant de l'huile de lin, du palmitate de vitamine A, du TPGS de vitamine E, et en phase aqueuse, de la vitamine B12 et des pycnogénols.

2. Solution de gouttes pour les yeux selon la revendication 1 **caractérisée en ce que** la solution liposomiale de gouttes pour les yeux contient en outre un système composé de 2-amino-2(hydroxyméthyl)propane-1,3-diol, qui agit en tant qu'agent de formation de sel de pycnogénols, soluble dans l'eau gazeuse, et d'un tampon régulateur de pH.

3. Solution de gouttes pour les yeux selon chacune des revendications précédentes, **caractérisée en ce qu'**elle est stérilisée par filtration à 0,2 µm, puisque la stérilisation à la vapeur atteinte par exposition de ladite solution à une vapeur saturée à des températures élevées à 121 °C pendant 15 minutes à 1·10⁵ Pa (1 atm) détruit tous les composants des liposomes.

4. Solution de gouttes pour les yeux selon chacune des revendications précédentes, **caractérisée en ce que** les gouttes liposomiales pour les yeux exercent un effet de protection contre les rayons UVA UVB (UVA 315 à 400 nm - UVB 315 à 280), une telle propriété étant principalement liée aux liposomes (plus TPGS de vitamine E) et secondairement à la vitamine B12 et aux pycnogénols en phase aqueuse.

5. Solution de gouttes pour les yeux selon chacune des revendications 2 à 4, **caractérisée en ce qu'**elle a la composition suivante :
| | |
|---|---|
| Ingrédients | **% p/p** |
| Phospholipide S80 | 0,250/**0,750** |
| Huile de lin | 0,025/**0,075** |
| Palmitate de vitamine A | 0,005/**0**,**015** |
| TPGS de vitamine E à l'intérieur des liposomes | 0,0125/**0,0375** |
| Pycnogénol | 0,0085/**0,0255** |
| Tris (2-amino-2(hydroxyméthyl)propane-1,3-diol | 0,3 à 0,2/**0,4 à 0,3** |
| Vitamine B12 | 0,00175/**0,00525** |
| Sel de sodium hyaluronique (1,5 à 1,7 MDa) à l'extérieur des liposomes | 0,0750/**0,225** |
| Acide borique | 0,775 |
| Tétraborate de sodium décahydraté | 0,0600 |
| Chlorure de sodium | 0,2 à 0,15 |
| Eau distillée | 100 |
| Stérilisation par filtration à 0,2 µm (0,2 micron) | Oui |
| pH | 7,1 à 7,4 |
| Osmolalité mMOs/kg | 275 à 290 |

6. Solution de gouttes pour les yeux selon revendication 5, dans laquelle la composition est comme suit :
| Ingrédients | p/p |
|---|---|
| Phospholipide S80 | 0,500 |
| Huile de lin | 0,050 |
| Palmitate de vitamine A | 0,010 |
| Pycnogénol | 0,017 |
| Tris **(2-Amino-2(hydroxyméthyl)propane-1,3-diol** | 0,3 à 0,2 |
| Vitamine B12 | 0,0035 |
| Sel de sodium hyaluronique (1,5 à 1,7 MDa) à l'extérieur des liposomes | 0,150 |
| TPGS de vitamine E à l'extérieur des liposomes | 0,025 |
| Acide borique | 0,775 |
| Tétraborate de sodium décahydraté | 0,060 |
| Chlorure de sodium | 0,2 à 0,15 |
| Eau distillée | 100 |
| Stérilisation par filtration à 0,2 µm (0,2 micron) | Oui |
| pH | 7,1 à 7,4 |
| Osmolalité mMOs/kg | 275 à 290 |

7. Procédé de préparation d'une solution de gouttes pour les yeux pour une utilisation dans le traitement du syndrome des yeux secs, comprenant les étapes suivantes :
Première étape :
Préparation de liposomes portant des substances lipophiles :
- Solubiliser tous les ingrédients lipophiles, les phospholipides non hydrogénés, l'huile de lin, le palmitate de vitamine A plus TPGS de vitamine E par un solvant, habituellement de l'éthanol,
- Le solvant est éliminé sous vide pour obtenir une poudre sèche,
- La poudre est dispersée dans un tampon de borate et mélangée par un mélangeur mécanique, une solution de grands liposomes est obtenue, qui pourraient être définis comme étant des pro-liposomes ;
- La solution de ces grands liposomes est ensuite extrudée à haute pression, entre 600/1 000 bar ;
- La procédure d'extrusion à haute pression est répétée plusieurs fois, entre 4 et 7, pour obtenir des liposomes de taille inférieure à 200 nm ;
Deuxième étape :
Préparation d'une solution aqueuse contenant des substances hydrophiles
- Solubiliser dans de l'eau distillée des pycnogénols, du Tris, de la vitamine B12, de l'acide hyaluronique et un tampon de borate ;
dans laquelle la concentration de substances lipophiles dans la première étape et la concentration de substances hydrophiles dans la deuxième étape sont telles qu'elles permettent, ajoutées l'une à l'autre en un volume 1:1, d'obtenir la solution ayant la concentration souhaitée ;
- Une solution colorée limpide et rouge est obtenue ;
Troisième étape :
Préparation de gouttes stériles finales pour les yeux :
Une quantité « X » de solution de liposomes, préparée à la première étape, est ajoutée à une même quantité « X » de solution aqueuse colorée, préparée à la deuxième étape, obtenant une quantité de « 2X » d'une solution de gouttes standard finales pour les yeux de la formule selon la revendication 5, et
la solution de gouttes standard finales pour les yeux est stérilisée par filtration à une filtration de 0,2 µm (0,2 micron).

8. Solution de gouttes pour les yeux selon chacune des revendications 4 et 5 pour une utilisation dans le traitement du syndrome des yeux secs **caractérisée en ce qu'**elle est utilisée en tant que protection contre les rayons UVA/UVB.

9. Solution de gouttes pour les yeux selon chacune des revendications 1 à 6 précédentes pour une utilisation dans le traitement du syndrome des yeux secs **caractérisée en ce qu'**elle est destinée à réduire les effets négatifs du syndrome des yeux secs.

10. Solution de gouttes pour les yeux obtenue par le procédé selon la revendication 7 pour une utilisation dans le traitement du syndrome des yeux secs **caractérisée en ce qu'**elle est utilisée en tant que protection contre les rayons UVA/UVB.
